(19) Europäisches Patentamt
European Patent Office
Office européen des brevets

(11) Publication number: **0 022 371**
**B1**

(12) # EUROPEAN PATENT SPECIFICATION

(45) Date of publication of patent specification: **19.01.83**

(21) Application number: **80302305.0**

(22) Date of filing: **08.07.80**

(51) Int. Cl.³: **C 07 D 295/08,**
C 07 D 211/14,
C 07 D 211/26,
C 07 D 211/62,
A 61 K 31/395

(54) **Substituted piperazines and piperidines and pharmaceutical compositions containing them.**

(30) Priority: **09.07.79 US 55936**
**05.05.80 US 146846**

(43) Date of publication of application:
**14.01.81 Bulletin 81/2**

(45) Publication of the grant of the patent:
**19.01.83 Bulletin 83/3**

(84) Designated Contracting States:
**AT BE CH DE FR GB IT LI LU NL SE**

(56) References cited:
**BE - A - 570 307**
**DE - A - 2 835 369**
**FR - M - 5 012**

**CHEMICAL ABSTRACT, vol. 89, no. 7, August 14, 1978, page 562 ref 59771t Columbus, Ohio, US**

(73) Proprietor: **PFIZER INC.**
**235 East 42nd Street**
**New York, N.Y. 10017 (US)**

(72) Inventor: **Kraska, Allen Richard**
**20 Joval Street**
**East Lyme, New London Connecticut (US)**
Inventor: **Lombardino, Joseph George**
**13 Laurel Hill Drive**
**Niantic, New London Connecticut (US)**

(74) Representative: **Graham, Philip Colin Christison et al,**
**Pfizer Limited Ramsgate Road**
**Sandwich, Kent CT13 9NJ (GB)**

**EP 0 022 371 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Substituted piperazines and piperidines and pharmaceutical compositions containing them

This invention relates to novel substituted piperazine and piperidine derivatives useful as immune regulants.

A number of compounds have been known in the art to be useful as antiinflammatory agents, for example the corticosteriods, phenylbutazone, indomethacin and various 3,4-dihydro-4-oxo-2H-1,2-benzothiazine-4-carboxamide-1,1-dioxides, such as those disclosed in United States Patent No. 3,591,584. Accordingly, these compounds have been of therapeutic value in the treatment of arthritic and other inflammatory conditions such as rheumatoid arthritis. Such conditions have also been treated by administration of immunoregulatory agents, such as levamisole, as described for example in Arthritis Rheumatism, *20* 1445 (1977) and Lancet, *1*, 393 (1976).

It is also known that biological vaccines such as *Corynebacterium pavrum* and BCG, a viable strain of *Myobacterium bovis*, have utility as immune stimulants of the reticulo-endothelial system and are thereby capable of increasing the resistance of a warm-blooded animal to tumors. However, the use of these agents has been restricted by hepatic-renal toxicity, granuloma formation, neutropenia and inconsistent therapeutic effects. Accordingly, it has been of continuing interest to develop non-biological, systemically active immune stimulants for use in increasing the resistance of a host to tumors. For discussions of the stimulation of cell-mediated immunity and antitumoral activity, see Herberman, Adv. Cancer Res., *19*, 207 (1971), Jordan and Merigan, Ann. Rev. Pharmacol., *15*, 157 (1975), Levy and Wheelock, Adv. Cancer Res., *20*, 131 (1972) and Sinkovics, Post Graduate Medicine, *59*, 110 (1976).

The present invention relates to novel substituted piperazines and piperidines having immune regulant activity. More specifically, the compounds of the present invention are those of Formula I:

$$R_1—O—CH_2—\underset{\underset{OH}{|}}{CH}—CH_2—X \qquad I$$

and the pharmaceutically acceptable acid addition salts thereof, wherein X is

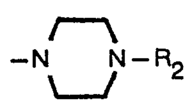
or
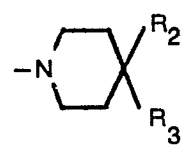

wherein $R_2$ is hydrogen, alkyl of 1 to 3 carbon atoms, benzyl, monosubstituted benzyl, phenyl or monosubstituted phenyl, wherein said substituents are chloro, bromo, fluoro, alkyl of 1 to 3 carbon atoms or alkoxy of 1 to 3 carbon atoms; $R_3$ is hydrogen, methyl, $—CH_2NH_2$ or $—CONH_2$; and $R_1$ is n-alkyl of 10 to 20 carbon atoms, benzyl or monosubstituted benzyl, said substituent being chloro, bromo, fluoro, alkyl of 1 to 3 carbon atoms or alkoxy of 1 to 3 carbon atoms, with the proviso that $R_1$ is benzyl or monosubstituted benzyl only when X is

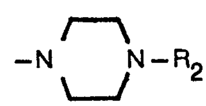

and $R_2$ is benzyl or monosubstituted benzyl.

Compounds of the formula:

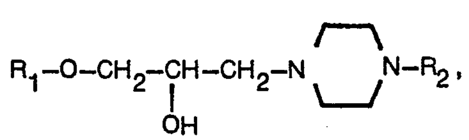

in which $R_1$ is benzyl or substituted benzyl, and $R_2$ is phenyl, are disclosed in published Japanese patent application (Kokai) 7821,132, referred to in Chemical Abstracts, vol. 89, no. 7, 14 August 1978, page 562, ref. 59771t, where they are described as having central nerve depressing activity.

One group of compounds of interest is that wherein X is $R_2$-substituted-piperazino. Preferred compounds are those wherein $R_1$ is from 16 to 20 carbon atoms, most preferably 16 or 18 carbon atoms, or benzyl. Preferred substituents for $R_2$ are benzyl and monosubstituted benzyl, for example 4-chlorobenzyl, 4-fluorobenzyl, 4-methylbenzyl, 4-methoxybenzyl, phenyl and monosubstituted phenyl for example 3-trifluoromethylphenyl. An especially preferred group for $R_2$ is benzyl, especially where $R_1$

is n-alkyl of 16 carbon atoms or benzyl. A further preferred group of compounds are those where $R_2$ is hydrogen, especially where $R_1$ is n-alkyl of 16 carbon atoms.

A further group of compounds of interest is that wherein X is ($R_2$, $R_3$)-substituted-piperidino. Preferred compounds are those wherein $R_1$ is of 16 to 20 carbon atoms, most preferably 16 or 18 carbon atoms. Preferred substituents for $R_2$ are hydrogen and phenyl, including those compounds wherein $R_3$ is either hydrogen, methyl, or $-CH_2NH_2$ or $-CONH_2$.

The present invention also includes pharmaceutical compositions containing the novel compounds of formula I together with a pharmaceutically-acceptable carrier or diluent. Preferred pharmaceutical compositions are those containing the preferred novel compounds of formula I as set forth hereinabove.

Also embraced by the present invention is a method of immune regulation in a host which comprises administering to the host an effective immune regulant amount of a compound of formula I, preferably selected from the preferred compounds of formula I described hereinabove.

The novel compounds of formula I are readily prepared by reaction of an appropriate 2,3-epoxypropyl-n-alkyl ether or 2,3-epoxypropyl-benzyl ether with a suitable substituted piperidine or piperazine. The 2,3-epoxypropyl-n-alkyl ether or 2,3-epoxypropyl-benzyl ether is prepared by reaction of an appropriate n-alkyl alcohol or benzyl alcohol with an allyl halide, preferably allyl chloride or bromide. The reaction is generally conducted in the presence of an alkali metal hydride such as sodium hydride in an inert organic solvent, such as dimethyl formamide, at temperatures from 50°C to 120°C, preferably 75—100°C. The allyl-n-alkyl ether or allyl benzyl ether formed in this reaction is then oxidized to the corresponding 2,3-epoxypropyl-n-alkyl ether or 2,3-epoxypropyl-benzyl ether by conventional oxidative methods using, for example, a peroxy acid such as perbenzoic acid, m-chloroperbenzoic acid and the like. The reaction is generally conducted in an inert organic solvent, such as methylene chloride, chloroform, and the like at a temperature of 10—50°C, preferably at about room temperature.

The 2,3-epoxypropyl-n-alkyl ether or 2,3-epoxypropyl-benzyl ether is reacted with an appropriate 1-$R_2$-substituted-piperazine or a 4,4-$R_2R_3$-substituted -piperidine. The reaction is effected by heating the reactants at a temperature of 75°C to 250°C, preferably at 150 to 200°C, preferably employing a small excess of the substituted piperazine or piperidine. The time necessary for completion of the reaction will vary with the temperature employed but is generally from 15 minutes to 2 hours at the preferred temperatures in the range 150 to 200°C. The reaction is preferably conducted without the addition of a solvent, but if desired, a reaction inert solvent, such as dimethyl formamide and the like, may be employed.

For preparation of the $R_2$-substituted-piperazino compounds of this invention where $R_1$ is n-alkyl of 10 to 20 carbon atoms an alternative and convenient method of preparation is to react the 2,3-epoxypropyl-n-alkyl ether with the readily available 1-benzyl-piperazine to form 1-benzyl-4-(2-hydroxy-3-n-alkoxypropyl)-piperazine, in accord with the reaction procedures described above. The 1-benzyl group may then be removed by hydrogenolysis, for example using a palladium on carbon catalyst and hydrogen. The reaction is generally conducted at a temperature between 10—50°C, preferably at about room temperature, in an inert organic solvent, such as an ether like tetrahydrofuran, dioxane or dimethoxyethane, or an n-alkanol of 1 to 4 carbon atoms, particularly methanol or ethanol. The 1-(2-hydroxy-3-n-alkoxypropyl)-piperazine (i.e. where $R_2$ is hydrogen) formed in this manner can then be further reacted to form those compounds having other $R_2$ groups. Thus, for example, the 1-(2-hydroxy-3-n-alkoxypropyl)-piperazine may be reacted with an appropriately substituted benzyl halide in the presence of a base, for example a trialkyl amine such as triethylamine. The reaction is generally conducted in an inert organic solvent, for example an ether such as tetrahydrofuran, dioxane, dimethoxyethane and the like, at temperatures in the range of 50—150°C, preferably at reflux temperature of the solvent. Compounds where $R_2$ is alkyl may be prepared by reaction of 1-(2-hydroxy-3-n-alkoxypropyl)-piperazine with an appropriate alkyl halide using the reaction conditions described above for reaction of a benzyl halide.

Preparation of the ($R_2$,$R_3$)-substituted-piperidino compounds of this invention wherein $R_3$ is aminomethyl is effected by reaction of the appropriate 4-$R_2$-4-cyano-piperidine with the appropriate 2,3-epoxypropyl-n-alkyl ether as described above to form the 4-cyano-1-(2-hydroxy-3-n-alkoxypropyl)-4-$R_2$-piperidine. The 4-cyano group is then readily reduced to aminomethyl, for example with Raney nickel and hydrogen. The reaction is effected at a temperature of 40—140°C in an inert organic solvent such as an ether like tetrahydrofuran, dioxane, dimethoxyethane and similar solvents.

Preparation of the ($R_2$,$R_3$)-substituted-piperidine compounds wherein $R_3$ is a carboxamide group is effected by reaction of the appropriate 2,3-epoxypropyl-n-alkyl ether with an $R_2$-substituted-4-carboxamido-piperidine using the reaction methods described above. If desired, the 4-carboxamido-1-(2-hydroxy-3-n-alkoxypropyl)-piperidine may be employed as an intermediate for the preparation of the corresponding 4-aminomethyl-substituted compounds by reduction of the 4-carboxamido group with a metal hydride, such as lithium aluminum hydride or sodium bis(2-methoxyethoxy)aluminum hydride and the like, generally at a temperature between 25—100°C in an inert organic solvent such as benzene, toluene, xylene and the like.

The pharmaceutically acceptable acid addition salts of the novel substituted piperazines and

piperidines of formula I are readily prepared by contacting the free base with the appropriate mineral or organic acid in either aqueous solution or in a suitable organic solvent, for example a lower alkanol having from 1 to 6 carbon atoms. The solid salt may then be obtained by precipitation or by evaporation of the solvent. The pharmaceutically acceptable acid addition salts of this invention include, but are not limited to, the hydrochloride, hydrobromide, hydroiodide, sulfate, bisulfate, nitrate, phosphate, acetate, lactate, maleate, fumarate, citrate, tartrate, succinate, gluconate, methanesulfonate, and the like.

The novel compounds of this invention and their pharmaceutically acceptable acid addition salts are useful regulants of the immune response in warm-blooded animals. These compounds are therefore useful in the treatment of conditions such as rheumatoid arthritis and other diseases associated with immune deficiency and accompanied by inflammation. Like the known compound Levamisole, a compound employed for the treatment of rheumatoid arthritis, the compounds of the present invention act to regulate the immune response of the subject and thereby alleviate the underlying immune disorder by maintaining immune competence. In addition, the activity of the present novel compounds as immune regulants makes them useful in maintaining the immune response of a warm-blooded animal to increase the resistance of the subject to tumors, the compounds acting to stimulate the natural immune system of the subject to reject tumors.

The present invention therefore also embraces a method of immune regulation in a warm-blooded animal by administering to the subject of an effective immune regulant amount of a compound of the present invention of formula I or a pharmaceutically acceptable acid addition salt thereof. In accord with this method, the compounds of the present invention may be administered to the subject in need of treatment by conventional routes, such as orally or parenterally, dosages in the range of 0.10 to 75 mg/kg body weight of the subject per day, preferably 0.15 to 15 mg/kg body weight per day being suitable. However, the optimum dosage for the individual subject being treated will be determined by the person responsible for treatment, generally smaller doses being administered initially and thereafter gradual increments made to determine the most suitable dosage. This will vary according to the particular compound employed and with the subject being treated. In this regard, the immune competence of the subject being treated may be monitored following administration using conventional techniques employed in the art and the response of the subject determined.

The compounds of this invention may be used in pharmaceutical preparations containing the compound or a pharmaceutically acceptable acid addition salt thereof in combination with a pharmaceutically acceptable carrier or diluent. Suitable pharmaceutically acceptable carriers include inert solid fillers or diluents and sterile aqueous or organic solutions. The active compound will be present in such pharmaceutical compositions in amounts sufficient to provide the desired dosage amount in the range described above. Thus, for oral administration the compounds may be combined with a suitable solid or liquid carrier or diluent to form capsules, tablets, powders, syrups, solutions, suspensions and the like. The pharmaceutical compositions may, if desired, contain additional components such as flavorants, sweetners, excipients and the like. For parenteral administration the compounds may be combined with sterile aqueous or organic media to form injectable solutions or suspensions. For example, solutions of the compounds of formula I in sesame or peanut oil, aqueous propylene glycol and the like may be used, as well as aqueous solutions or water soluble pharmaceutically acceptable acid addition salts of the compounds. The injectable solutions prepared in this manner may then be administered intraveneously, interperitoneally, subcutaneously or intramuscularly, with intraveneous and interperitoneal administration being preferred.

The immune regulant activity of the compounds of the present invention may be determined by such standard pharmacological tests as the stimulation *in vitro* of lymphocyte proliferation of murine thymus cells cultured in the presence of Concanavalin A (Con A) employing the general evaluation procedure of V. J. Merluzzi et al., see Journal of Clinical and Experimental Immunology, Volume 22, page 486 (1975). In this study, three different levels of peak lymphocyte stimulation assay (LSA) activity were established for the compounds undergoing evaluation, viz., those equal to Con A alone; those superior to Con A activity but less than levamisole, the standard compound of choice in this area; and those having an activity equal to levamisole. Compounds are considered to be active for the present purposes if they are superior to Concanavalin A.

The immune regulant activity of the compounds of the present invention may also be determined by an assessment of tumor rejection in, for example, the sarcoma 180 J model. In this test, the increased life span (% ILS) is determined for a group of female CD—1 mice (20—25 g). The mice receive $10^6$ S—180 J cells which are 5 to 8 days old, by intraperitoneal administration. On the day following tumor inoculation the mice receive 0.1 ml of the test compound formulated in Tween-glycerol at the desired dose level and are then observed until death or for 40 days, whichever occurs first. The increased life span is then determined from the ratio of the mean survival time of drug treated mice to the mean survival time of untreated control group mice.

A further test for this purpose is the CaD2 adenocarcinoma model. In this model, a group of B6D2F1 female mice are implanted subcutaneously in the side with 1 $mm^2$ fragments of $CaD_2$ mammary adenocarcinoma. After 15 days, the primary tumor is surgically removed. The test compound is then administered orally at the desired dose level and the animals are monitored until death or for 100 days, whichever occurs first. The increased life span is then determined from the ratio of the mean

survival time of drug treated mice to the mean survival time of untreated controlled group mice.

The present invention is illustrated by the following examples. However, it should be understood that the invention is not limited to the specific details of these examples.

Example 1

*Allyl-n-hexadecylether:* *n*-Hexadecanol (24.2 g, 0.1 mol) was added to a suspension of sodium hydride (9.6 g of a 50% dispersion in mineral oil, 0.2 mol) in dimethylformamide (200 ml) and heated to 50°C for 30 minutes. Allyl bromide (24.2 g, 0.2 mol) was then added and the mixture heated to 90°C for 5 hours. The reaction was cooled, diluted with a solution of saturated sodium chloride (300 ml), and extracted with ether (2 × 300 ml). The combined ether extracts were washed with a saturated solution of sodium chloride (200 ml), filtered, dried over magnesium sulfate, treated with activated charcoal, filtered, and evaporated under reduced pressure to an oil. The resulting oil was purified by absorbing it on silica gel, placing the silica gel in a sintered glass filter, and eluting the product off with hexane (7 × 200 ml) followed by toluene (4 × 200 ml). Concentration yielded the pure ether (14 g, 50% yield): NMR ($CDCl_3$) 3.40 (t, 2, $—CH_2—O—CH_2CH{=}CH_2$), 3.92 (d, 2, $—O—CH_2CH{=}CH_2$), 4.95—5.38 (m, 2, $=CH_2$) and 5.58—6.20 (m, 1, $—CH{=}CH_2$).

Example 2

*2,3-Epoxypropyl-n-hexadecylether:* Allyl-n-hexadecylether (37 g, 0.13 mol) was dissolved in methylene chloride (200 ml) and treated with *m*-chloro-perbenzoic acid (31 g, 0.18 mol) at room temperature for 16 hours. More *m*-chloroperbenzoic acid (2.0 g, 0.01 mol) was added and the mixture stirred an additional 16 hours. The mixture was then filtered, treated with a saturated solution of sodium sulfite, and stirred for 2 hours. The methylene chloride layer was separated, washed with a saturated solution of sodium bicarbonate (4 × 500 ml) and water (500 ml), dried over magnesium sulfate, filtered and concentrated under reduced pressure to the desired epoxide, which solidified on cooling (35 g, 92% yield): mp 30—31.5°C; NMR ($CDCl_3$) 1.3 (S, 28, aliphatic methylene), 2.55 (d of d, 1, epoxide), 2.73 (t, 1, epoxide) and 3.03 (m, 1, epoxide).

Example 3

*1-Benzyl-4-(2-hydroxy-3-n-hexadecyloxypropyl)piperazine*

2,3-Epoxypropyl-n-hexadecylether (2.0 g, 0.0067 mol) and 1-benzylpiperazine (2.03 g, 0.011 mol) were combined and heated to 180°C for 20 minutes. The resulting mixture was dissolved in acetone and cooled in a dry ice/acetone bath. The solids that formed were filtered, dissolved in ether, and treated with gaseous hydrochloric acid. The desired amine hydrochloride was obtained by concentrating the ether solution and recrystallizing the resulting solid from isopropyl alcohol (0.8 g, 13% yield): mp 237—238°C; NMR [$(CD_3)_2SO$] 1.32 (S, 28, aliphatic methylene) and 4.55 (S, 2, $Ph—CH_2—N$);

*Anal:* Calcd for $C_{30}H_{54}O_2N_2.2HCl.1/2\ H_2O$: C, 64.72; H, 10.32; N, 5.03;
Found: C, 64.87; H, 10.22; N, 5.00.

Example 4

*1-(2-Hydroxy-3-n-hexadecyloxypropyl)-piperazine*

1-Benzyl-4-(2-hydroxy-3-n-hexadecyloxypropyl)-piperazine hydrochloride (8 g, 0.0146 mol) was dissolved in toluene (100 ml), methanol (100 ml) and tetrahydrofuran (700 ml) and divided into three 300 ml portions. Each portion was hydrogenated overnight at room temperature on a Parr shaker using a 10% palladium on carbon catalyst (2 g) and 50 p.s.i.g. hydrogen. The catalyst was filtered off and the filtrate evaporated under reduced pressure to a solid, which was triturated under acetone and filtered to give a total yield of product of 2.9 g (43%), mp 230°C (dec).

*Anal:* Calcd for $C_{23}H_{48}O_2N_2.2HCl$: C, 60.37; H, 11.01; N, 6.12.
Found: C, 60.26; H, 10.71; N, 5.97.

Example 5

*1-(3,4-Dichlorobenzyl)-4-(2-hydroxy-3-n-hexadecyloxypropyl)-piperazine*

1-1(2-Hydroxy-3-n-hexadecyloxypropyl)-piperazine (0.7 g, 0.0015 mol), 3,4-dichlorobenzylchloride (0.328 g, 0.00168 mol) and triethylamine (1.39 ml, 0.01 mol) were refluxed in tetrahydrofuran (50 ml) for 16 hours. The mixture was cooled, diluted with water (200 ml) and extracted with ether (200 ml). The ether extract was washed with water (2 × 100 ml), dried over magnesium sulfate, filtered and concentrated under reduced pressure. The resulting oil was purified by silica gel chromatography (eluted with 5% ethanol in toluene), converted to the hydrochloride salt and recrystallized from hot isopropyl alcohol to give pure product (0.35 g, 38% yield): mp 236—237°C.

*Anal:* Calcd for $C_{30}H_{52}O_2N_2Cl_2.2HCl$: C, 58.44; H, 8.83; N, 4.54.
Found: C, 58.06; H, 8.90; N, 4.48.

Example 6

*1-Methyl-4-(2-hydroxy-n-hexadecyloxypropyl)-piperazine*

Following the procedures of Example 3, 2,3-epoxypropyl-n-hexadecylether was reacted with 1-methylpiperazine to form 1-methyl-4-(2-hydroxy-n-hexadecyloxypropyl)-piperazine, mp 209—211°C.

*Anal:* Calcd for $C_{24}H_{50}O_2N_2$.2HCl.1/2$H_2O$: C, 59.98; H, 11.12; N, 5.82.
Found: C, 59.80; H, 10.88; N, 5.70.

Example 7

*1-Benzyl-4-(2-hydroxy-3-n-octadecyloxypropyl)-piperazine*

Following the procedure of Examples 1 to 3, 2,3-epoxypropyl-n-octadecylether and 1-benzylpiperazine were reacted to form 1-benzyl-4-(2-hydroxy-3-n-octadecyloxypropyl)-piperazine, mp 235—236°C.

*Anal:* Calcd for $C_{32}H_{58}O_2N_2$.2HCl.3/4$H_2O$: C, 65.23; H, 10.52; N, 4.75.
Found: C, 65.39; H, 10.26; N, 4.75.

Example 8

*1-(4-Chlorobenzyl)-4-(2-hydroxy-3-n-hexadecyloxypropyl)-piperazine*

Following the procedure of Example 5, 1-(2-hydroxy-3-n-hexadecyloxypropyl)-piperazine was reacted with 4-chlorobenzylchloride to form 1-(4-chlorobenzyl)-4-(2-hydroxy-3-n-hexadecyloxypropyl)-piperazine, mp 237—239°C.

*Anal:* Calcd for $C_{30}H_{53}O_2N_2Cl$.2HCl.1/4$H_2O$: C, 61.42; H, 9.53; N, 4.78.
Found: C, 61.39; H, 9.10; N, 4.75.

Example 9

*1-(4-Methylbenzyl)-4-(2-hydroxy-3-n-hexadecyloxypropyl)-piperazine*

Following the procedure of Example 5, 1-(2-hydroxy-3-n-hexadecyloxypropyl)-piperazine was reacted with 4-methylbenzyl chloride to form 1-(4-methylbenzyl)-4-(2-hydroxy-3-n-hexadecyloxypropyl)-piperazine, mp 238—239°C.

*Anal:* Calcd for $C_{31}H_{56}O_2N_2$.2HCl.3/4HCl: C, 64.73; H, 10.42; N, 4.87.
Found: C, 64.75; H, 10.36; N, 4.96.

Example 10

*1-(3-Trifluoromethylbenzyl)-4-(2-hydroxy-3-n-hexadecyloxypropyl)-piperazine*

Following the procedure of Example 5, 1-(2-hydroxy-3-n-hexadecyloxypropyl)-piperazine was reacted with 3-(trifluoromethyl)benzyl chloride to form 1-(3-trifluoromethylbenzyl)-4-(2-hydroxy-3-n-hexadecyloxypropyl)-piperazine, mp 133—135°C.

*Anal:* Calcd for $C_{30}H_{51}O_2N_2F_3$.HCl: C, 63.75; H, 9.27; N, 4.96.
Found: C, 63.62; H, 9.13; N, 4.87.

Example 11

*1-(4-Fluorobenzyl)-4-(2-hydroxy-3-n-hexadecyloxypropyl)-piperazine*

Following the procedure of Example 5, 1-(2-hydroxy-3-n-hexadecyloxypropyl)-piperazine was reacted with 4-fluorobenzyl chloride to form 1-(4-fluorobenzyl)-4-(2-hydroxy-3-n-hexadecyloxypropyl)-piperazine, mp 242°C (dec).

*Anal:* Calcd for $C_{30}H_{53}O_2N_2F$.2HCl.1/4$H_2O$: C, 63.20; H, 9.81; N, 4.91.

Example 12

*1-(4-Methoxybenzyl)-4-(2-hydroxy-3-n-hexadecyloxypropyl)-piperazine*

Following the procedure of Example 5, 1-(2-hydroxy-3-n-hexadecyloxypropyl)-piperazine was reacted with 4-methoxybenzyl chloride to form 1-(4-methoxybenzyl)-4-(2-hydroxy-3-n-hexadecyloxypropyl)-piperazine, mp 233—245°C.

*Anal:* Calcd for $C_{31}H_{56}O_3N_2$.2HCl.1/4$H_2O$: C, 63.95; H, 10.13; N, 4.81.
Found: C, 63.74; H, 9.88; N, 4.78.

Example 13

*1-Benzyl-4-(2-hydroxy-3-n-dodecyloxypropyl)-piperazine*

Following the procedures of Examples 1 to 3, 2,3-epoxypropyl-n-dodecylether and 1-benzylpiperazine were reacted to form 1-benzyl-4-(2-hydroxy-3-n-dodecyloxypropyl)-piperazine, mp 239°C (dec).

*Anal:* Calcd for $C_{26}H_{46}O_2N_2.2HCl$: C, 63.53; H, 9.84; N, 5.70.
Found: C, 63.88; H, 9.23; N, 5.63.

Example 14

*1-(4-Fluorobenzyl)-4-(2-hydroxy-3-n-dodecyloxypropyl)-piperazine*

Following the procedures of Examples 4 and 5, 1-benzyl-4-(2-hydroxy-3-n-dodecyloxypropyl)-piperazine was converted to 1-(2-hydroxy-3-n-dodecyloxypropyl)-piperazine by hydrogenolysis using a palladium on carbon catalyst. The hydrogenolysis product was reacted with 4-fluorobenzyl chloride to form 1-(4-fluorobenzyl)-4-(2-hydroxy-3-n-dodecyloxypropyl)-piperazine, mp 243—245°C.

*Anal:* Calcd for $C_{26}H_{45}O_2N_2F.2HCl.H_2O$: C, 59.19; H, 9.36; N, 5.31.
Found: C, 59.35; H, 9.25; N, 5.79.

Example 15

*1-Phenyl-4-(2-hydroxy-3-n-hexadecyloxypropyl)-piperazine*

Following the procedure of Example 3, 2,3-epoxypropyl-n-hexadecylether and 1-phenylpiperazine were reacted to form 1-phenyl-4-(2-hydroxy-3-n-hexadecyloxypropyl)-piperazine, mp 195—196°C.

*Anal:* Calcd for $C_{29}H_{52}O_2N_2.2HCl$: C, 65.27; H, 10.20; N, 5.25.
Found: C, 65.58; H, 9.99; N, 5.45.

Example 16

*1-Benzyl-4-(2-hydroxy-3-benzyloxypropyl)-piperazine Dihydrochloride*

Sodium hydride (4.8 g, 0.2 mol, 50% dispersion in mineral oil) was added under nitrogen at room temperature to benzyl alcohol (21.6 g., 0.2 mol) in 150 ml. of dry dimethylformamide. The suspension was stirred at room temperature for 30 minutes and then heated at 60°C. for 30 minutes. Allyl bromide (24.2 g., 0.2 mol) was added to the suspension dropwise at 50°C. with stirring. The reaction mixture was heated at 90°C. for five and a half hours under nitrogen, cooled and stirred at room temperature overnight. The dimethylformamide was removed on a rotary evaporator and the resulting oil was extracted with ether, dried over sodium sulfate and the ether evaporated off. The resulting oil was purified on a silica gel column using hexane (500 ml) to remove the mineral oil, followed by methylene chloride (750 ml) to elute the desired product. Evaporation of the solvent yielded allylbenzyl ether as a pale yellow liquid, 16 g., 54% yield.

Allylbenzyl ether (1.50 g., 0.01 mol.), was dissolved in 36 ml. methylene chloride and treated with m-chloroperbenzoic acid (4.10 g, 0.02 mole) at room temperature for 17 hours. The mixture was then filtered and 100 ml of 5% sodium sulfite solution was added to the filtrate, followed by addition of 50 ml saturated sodium bicarbonate. The methylene chloride layer was separated, washed three times with sodium sulfate and once with water and dried over sodium sulfate. Removal of the methylene chloride under vacuum yielded the 2,3-epoxypropyl-benzyl ether as a pale gold oil, 1.44 g., 87% yield.

2,3-Epoxypropyl-benzyl ether (1.22 g., 0.0074 mole) and 1-benzylpiperazine (1.31 g, 0.0074 mole) were combined and heated under nitrogen at 150°C. for two and a half hours. The reaction mixture was diluted to 400 ml. with diethyl ether and anhydrous hydrogen chloride gas was bubbled through the solution while the solution was stirred and cooled. The solids formed were filtered, washed with diethyl ether and dried over phosphorus pentoxide under vacuum. The resulting solids were dissolved in 75 ml refluxing isopropanol, the solution filtered, evaporated down to a volume of 30 ml and allowed to cool slowly. The resulting crystals were broken up, filtered, washed with cold isopropanol and vacuum dried over phosphorus pentoxide to give the title compound, 2.18 g., 71% yield, mp 215—216.5°C.

*Anal:* Calcd for $C_{21}H_{28}N_2O_2.2HCl$: C, 61.02; H, 7.31; N, 6.78.
Found: C, 60.48; H, 7.20; N, 6.75.

Example 17

*4-Cyano-1-(2-hydroxy-3-n-hexadecyloxypropyl)-4-phenylpiperidine:*

4-Cyano-4-phenylpiperidine (1.1 g, 0.0059 mol) and 2,3-epoxypropyl-n-hexadecylether (1.6 g, 0.0056 mol) were combined and heated to 185°C for 30 minutes. After cooling, ethyl acetate (20 ml) and acetonitrile (20 ml) were added, and the resulting solid was collected by filtration (1.6 g, 62% yield): mp 69—70°C; ir (KBr) 2227 $cm^{-1}$.

Example 18

*4-Aminomethyl-1-(2-hydroxy-3-n-hexadecyloxypropyl)-4-phenylpiperidine hydrochloride:*

4-Cyano-1-(2-hydroxy-3-n-hexadecyloxypropyl)-4-phenylpiperidine (1.5 g, 0.003 mol) was dissolved in a mixture of tetrahydrofuran (50 ml) and ethanol (3 ml) saturated with ammonia. Raney-nickel (0.8 g) was added, and the mixture was hydrogenated at 50 psi ($H_2$) for 2 hours. The mixture was then filtered and concentrated under reduced pressure to a waxy solid. This solid was dissolved in methylene chloride, treated with gaseous hydrochloric acid and again concentrated to a solid. The pure product was obtained by recrystallization from isopropyl alcohol (1.0 g, 59% yield): mp 227—228°C.

*Anal:* Calcd for $C_{31}H_{56}O_2N_2$.2HCl: C, 66.29; H, 10.41; N, 4.99.
Found: C, 66.18; H, 10.40; N, 5.18.

Example 19

*4-Aminomethyl-1-(2-hydroxy-3-n-octadecyloxypropyl)-4-phenylpiperidine:*

Following the procedures of Example 17 and 18, 4-cyano-4-phenylpiperidine was reacted with 2,3-epoxypropyl-octadecylether and reduced to form 4-aminomethyl-1-(2-hydroxy-3-n-octadecyloxypropyl)-4-phenylpiperidine, mp 223—224°C.

*Anal:* Calcd for $C_{33}H_{60}O_2N_2$.2HCl.2.25$H_2$: C, 62.88; H, 10.63, N, 4.44.
Found: C, 62.96; H, 10.25, N, 4.56.

Example 20

*4-Carboxamido-1-(2-hydroxy-3-n-hexadecyloxypropyl)piperidine:*

Isonipecotamide (0.7 g, 0.0055 mol) and 2,3-epoxypropyl-n-hexadecylether (1.49 g, 0.005 mol) were combined and heated to 180°C for 20 minutes. The reaction mixture was cooled, ethyl acetate (20 ml) added, and the resulting solids isolated by filtration. Recrystallization from hot ethyl acetate (15 ml) gave pure product (1.6 g, 75% yield): mp 97—98°C; ir (KBr) 1653 $cm^{-1}$.

*Anal:* Calcd for $C_{25}H_{50}O_3N_2$: C, 70.37; H, 11.81; N, 6.57.
Found: C, 69.90; H, 11.42; N, 6.50.

Example 21

*4-Aminomethyl-1-(2-hydroxy-3-n-hexadecyloxypropyl)piperidine hydrochloride:*

4-Carboxamido-1-(2-hydroxy-3-n-hexadecyloxypropyl)piperidine (1.1 g, 0.0026 mol) was dissolved in toluene (20 ml) and a 70% solution of sodium bis-(2-methoxyethoxy)aluminum hydride (3.5 ml) was slowly added over 15 minutes. The reaction mixture was heated to 80°C for 16 hours and worked up by dropwise addition of ethyl acetate (10 ml) and then water (20 ml), followed by extraction with ether (50 ml). The ether extract was washed with water (2 × 50 ml), dried over magnesium sulfate, filtered, converted to the hydrochloride salt, and concentrated to a solid under reduced pressure. Pure product (0.25 g, 20% yield) was obtained by recrystallization from hot isopropyl alcohol/acetone (1/1): mp 202°C (dec).

*Anal:* Calcd for $C_{25}H_{52}O_2N_2$.2HCl.3/4$H_2O$: C, 60.16; H, 11.20; N, 5.61.
Found: C, 60.23; H, 10.53; N, 5.55.

Example 22

*1-(2-Hydroxy-3-n-hexadecyloxypropyl)-4-phenylpiperidine:*

4-Phenylpiperidine (1.8 g, 0.011 mol) and 2,3-epoxypropyl-n-hexadecylether (2.98 g, 0.01 mol) were combined and heated to 180°C for 30 minutes. The reaction mixture was cooled, acetone was added, and the mixture was stirred for 16 hours. The resulting solids were collected and recrystallized from hot acetone to give pure product (2.3 g, 50% yield): mp 64—65°C.

*Anal:* Calcd for $C_{30}H_{53}O_2N$: C, 78.37; H, 11.62; N, 3.05.
Found: C, 78.64; H, 11.42; N, 3.26.

Example 23

*1-(2-Hydroxy-3-n-octadecyloxypropyl)-4-phenylpiperidine:*

Following the procedure of Example 22, 2,3-epoxypropyl-n-octadecylether was reacted with 4-phenylpiperidine to give 1-(2-hydroxy-3-n-octadecyloxypropyl)-4-phenylpiperidine, mp 170—171°C.

*Anal:* Calcd for $C_{32}H_{57}O_2N$.HCl: C, 73.31; H, 11.15; N, 2.67.
Found: C, 73.57; H, 10.95; N, 2.54.

Example 24

*4-Methyl-1-(2-hydroxy-3-n-hexadecyloxypropyl)-piperidine:*

Following the procedure of Example 22, 2,3-epoxypropyl-n-hexadecylether was reacted with 4-methylpiperidine to form 4-methyl-1-(2-hydroxy-3-n-hexadecyloxypropyl)-piperidine, mp 84—85°C.

*Anal:* Calcd for $C_{25}H_{51}O_2N.HCl.1/4H_2O$: C, 68.45; H, 12.06; N, 3.19.
Found: C, 68.58; H, 12.28; N, 3.09.

Example 25

*Sarcoma 180J Model for Assessment of Tumor Rejection:*

Six female CD—1 mice (20—25 g) per group received $10^6$ S—180J cells which were 5 to 8 days old by intraperitoneal administration. On the day following tumor inoculation the mice received 0.1 ml of the test compound formulated in the fat emulsion vehicle Intralipid (Cutter Laboratories) by dissolving the drug in a minimum amount of alcohol and adding this solution to the Intralipid at the desired dose and were then observed until death or 40 days, whichever occurs first. Results are expressed as increased percent life span (%ILS), defined as follows:

$$\%ILS = \frac{(S - Sc)}{Sc} \times 100$$

Where S = Mean Survival time of Drug Treated Mice; and Sc = Mean Survival time of Control Mice. Results obtained by the above test procedure were as follows:

| Example | % ILS | Dose (mg/kg) |
|---|---|---|
| 3 | 64 | 1 |
| 4 | 73 | 15 |
| 6 | 54 | 64 |
| 10 | 28 | 15 |
| 11 | 25 | 75 |
| 13 | 89 | 75 |
| 14 | 26 | 0.6 |
| 16 | 32 | 15 |
| 18 | 78 | 16 |
| 19 | 88 | 15 |
| 20 | 115 | 16 |

Example 26

The immune regulant activity of the compounds of Examples 3, 5, 7, 9, 11, 13, 14, 15 and 16 was evaluated by determining their ability to stimulate, *in vitro* the lymphocyte proliferation of murine thymus cells cultured in the presence of Concanavalin A (Con. A) by employing the procedure of V. J. Merluzzi et. al., essentially as described in the *Journal of Clinical and Experimental Immunology,* Vol. 22, p. 486 (1975). The cells were derived from male C57B1/6 mice of from 6—8 weeks age, purchased from the Jackson Laboratories of Bar Harbor, Maine and the Con A was obtained from Sigma Chemicals of St. Louis, Missouri. Each cell culture (consisting of 0.10 ml thymus cells stock solution, 0.05 ml of Con A stock solution and 0.05 ml of drug solution) was performed in quadruplicate and cellular proliferation was measured, after 48 hours of incubation at 37°C, by pulsing each culture with $^3H$-thymidine (0.01 ml of specific activity 1.9 C/mM, obtained from Schwarz-Mann, Inc. of Orangeburg, N.Y.) and then determining the incorporation of $^3H$-thymidine into cellular desoxyribonucleic acid (DNA) by an assessment of radioactivity using a liquid scintillation counter. The results obtained in this manner are expressed quantitatively in terms of the average counts per minute (cpm) of $^3H$-thymidine incorporated at each drug level. On this basis, three different levels of peak activity were established in the present lymphocyte stimulation assay (LSA) and these are defined in the manner hereinafter indicated, viz., those levels equal to Con A alone (6,000 $\pm$ 300 cpm)

were assigned a negative value or score of zero; those superior (10,000 ± 700 cpm) to Con A activity but less than levamisole were scored as +; while those equal to levamisole (30,000 ± 900 cpm) were scored as ++ Minimum effective concentrations (MEC) were determined at activity levels with a score of +.

Peak Activity

| Example | Activity Level | Drug Concentration (μg/ml) |
|---|---|---|
| 3 | + | 0.10 |
| 5 | + | 1.0 |
| 7 | + | 0.01 |
| 9 | + | 0.01 |
| 11 | + | 0.30 |
| 13 | + | 0.30 |
| 14 | + | 0.10 |
| 15 | + | 0.30 |
| 16 | ++ | 0.04 |
| Levamisole | ++ | 28 |

Potency at + Activity Level

| Example | MEC (μg/ml) |
|---|---|
| 3 | 0.004 |
| 5 | 0.10 |
| 7 | <0.004 |
| 9 | <0.004 |
| 11 | 0.04 |
| 13 | <0.004 |
| 14 | 0.10 |
| 15 | 0.004 |
| 16 | ≪0.004 |
| Levamisole | 1.0 |

Example 27

*CaD2 Adenocarcinoma Model for Assessment of Antitumor Activity*

B6D2F1 female mice were implanted subcutaneously in the side with 1 mm.$^2$ fragments of CaD2 mammary adenocarcinoma. The tumor was allowed to grow and after 15 days surgery was performed to remove the primary tumor. The test compound was administered orally, and the animals were monitored until death or 100 days, whichever occurred first. The mean survival time was determined for control group and drug treated animals and the %ILS, defined as in Example 25, determined. The results obtained for 1-benzyl-4-(2-hydroxy-3-benzyloxypropyl)-piperazine (Example 16) were as follows:

| Dose (mg./kg.) | % ILS |
|---|---|
| 2.5 | 81 |
| 10 | 39 |
| 40 | 32 |

**Claims for the Contracting States: BE CH DE FR GB IT LI LU NL SE**

1. A compound of the formula

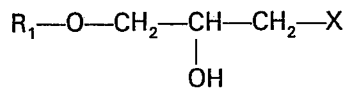

and the pharmaceutically acceptable acid addition salts thereof, wherein X is

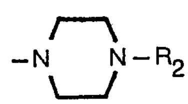
or
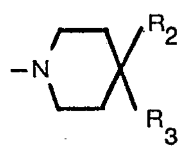

wherein

$R_2$ is hydrogen, alkyl of 1 to 3 carbon atoms, benzyl, monosubstituted benzyl, phenyl or monosubstituted phenyl, wherein said substituents are chloro, bromo, fluoro, alkyl of 1 to 3 carbon atoms or alkoxy of 1 to 3 carbon atoms; $R_3$ is hydrogen, methyl, —$CH_2NH_2$ or —$CONH_2$; and $R_1$ is n-alkyl of 10 to 20 carbon atoms, benzyl or monosubstituted benzyl, said substituent being chloro, bromo, fluoro, alkyl of 1 to 3 carbon atom or alkoxy of 1 to 3 carbon atoms, with the proviso that $R_1$ is benzyl or monosubstituted benzyl only when X is

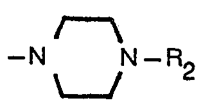

and $R_2$ is benzyl or monosubstituted benzyl.

2. A compound of claim 1 wherein X is

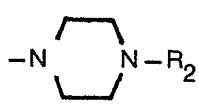

3. A compound of claim 2 wherein $R_2$ is benzyl, or monosubstituted benzyl.
4. A compound of Claim 3 wherein $R_1$ is n-alkyl of 16 or 18 carbon atoms or benzyl.
5. A compound of Claim 4 wherein $R_1$ and $R_2$ are each benzyl.
6. A compound of Claim 2 wherein $R_1$ is n-alkyl of 16 carbon atoms and $R_2$ is hydrogen.
7. A compound of Claim 1 wherein X is

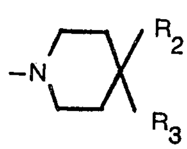

8. A compound of Claim 7 wherein $R_2$ is phenyl or hydrogen and $R_3$ is hydrogen, methyl, —$CH_2NH_2$ or —$CONH_2$.
9. A compound of Claim 8 wherein $R_1$ is n-alkyl of 16 or 18 carbon atoms.
10. A pharmaceutical composition comprising an immune-regulant effective amount of a compound of any preceding claim and a pharmaceutically acceptable carrier.

**Claims for the Contracting State: AT**

1. A process for the preparation of a compound of the formula

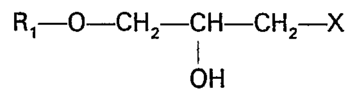

I

wherein

X is

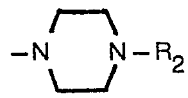

or

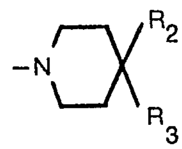

wherein

$R_2$ is hydrogen, alkyl of 1 to 3 carbon atoms, benzyl, monosubstituted benzyl, phenyl or monosubstituted phenyl; $R_3$ is hydrogen, methyl, $—CH_2NH_2$ or $—CONH_2$; and $R_1$ is n-alkyl of 10 to 20 carbon atoms, benzyl or monosubstituted benzyl; wherein said substituent are chloro, bromo, fluoro, alkyl of 1 to 3 carbon atoms or alkoxy of 1 to 3 carbon atoms; with the proviso that when $R_1$ is other than alkyl X is 1—$R_2$-piperazino and $R_2$ is benzyl or monosubstituted benzyl; characterized in that a compound of the formula

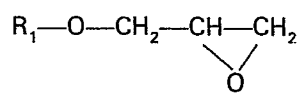

II

is heated in the presence of an appropriate 1—$R_2$-piperazine or 4—$R_2R_3$-piperidine of the formula HX, provided that when $R_3$ is $—CH_2NH_2$, a compound of formula II is heated with a 4—$R_2$-4-cyanopiperidine followed by reduction of the cyano group to $—CH_2NH_2$;

and, if desired, when $R_3$ is $—CONH_2$, reducing the compound of formula I to form the compound of formula I wherein $R_3$ is $—CH_2NH_2$;

and, if desired, subjecting the compound of formula I wherein X is 1-benzyl-piperazino and $R_1$ is n-alkyl, to hydrogenolysis to form the corresponding compound wherein X is piperazino;

and, if desired when X is piperazino and $R_1$ is n-alkyl, reacting the compound of formula I with an appropriate alkyl halide or substituted benzyl halide in the presence of a base to form a compound of formula I wherein X is 1—$R_2$-piperazino and $R_2$ is n-alkyl or substituted benzyl;

and, if desired, contacting the compound of formula I with a pharmaceutically acceptable acid to form an acid addition salt thereof.

2. A process according to Claim 1 wherein the compound of formula II is heated with the compound HX at a temperature of 75°C. to 250°C.

3. A process according to Claim 1 wherein said cyano group is reduced with Raney nickel and hydrogen at a temperature of 40°C. to 140°C.

4. A process according to Claim 1 wherein said $—CONH_2$ group is reduced with a metal hydride at a temperature of 25°C. to 100°C.

5. A process according to Claim 1 wherein said hydrogenolysis is conducted at a temperature of 10°C. to 50°C. using hydrogen and a palladium on carbon catalyst.

6. A process according to Claim 1 wherein said compound of formula I wherein X is piperazino is reacted with an n-alkyl halide or substituted benzyl halide at a temperature of 50°C. to 150°C. in the presence of a trialkylamine.

**Revendications pour les Etats contractants: BE CH LI DE FR GB IT LU NL SE**

1. Composé de formule

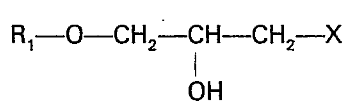

et ses sels d'addition d'acides pharmaceutiquement acceptables, formule dans laquelle X est un groupe

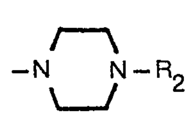

ou

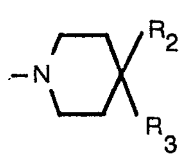

dans lequel $R_2$ est l'hydrogène, un radical alkyle ayant 1 à 3 atoms de carbone, benzyle, benzyle monosubstitué, phényle ou phényle monosubstitué, dont les substituants sont des radicaux chloro, bromo, fluoro, alkyle ayant 1 à 3 atoms de carbone ou alkoxy ayant 1 à 3 atomes de carbone, $R_3$ est l'hydrogène, un radical méthyle, $—CH_2NH_2$ ou $—CONH_2$; et $R_1$ est un radical n-alkyle de 10 à 20 atomes de carbone, benzyle ou benzyle monosubstitué dont le substituant est un radical chloro, bromo, fluoro, alkyle ayant 1 à 3 atomes de carbone ou alkoxy ayant 1 à 3 atomes de carbone, à condition que $R_1$ ne soit un radical benzyle ou benzyle monosubstitué que lorsque X est un groupe de formule

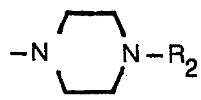 et

$R_2$ est un radical benzyle ou benzyle monosubstitué.

2. Composé suivant la revendication 1, dans lequel X est

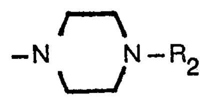

3. Composé suivant la revendication 2, dans lequel $R_2$ est un groupe benzyle ou benzyle monosubstitué.

4. Composé suivant la revendication 3, dans lequel $R_1$ est un radical n-alkyle de 16 ou 18 atomes de carbone ou un radical benzyle.

5. Composé suivant la revendication 4, dans lequel $R_1$ et $R_2$ sont chacun un radical benzyle.

6. Composé suivant la revendication 2, dans lequel $R_1$ est un radical n-alkyle de 16 atomes de carbone et $R_2$ est l'hydrogène.

7. Composé suivant la revendication 1, dans lequel X est

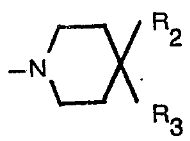

8. Composé suivant la revendication 7, dans lequel $R_2$ est le radical phényle ou l'hydrogène et $R_3$ est l'hydrogène ou le radical méthyle, $—CH_2NH_2$ ou $—CONH_2$.

9. Composé suivant la revendication 8, dans lequel $R_1$ est un radical n-alkyle de 16 ou 18 atomes de carbone.

10. Composition pharmaceutique comprenant une quantité à effet immunorégulateur d'un composé suivant l'une quelconque des revendications précédentes et un support pharmaceutiquement acceptable.

**Revendications pour l'Etat contractant: AT**

1. Procédé de préparation d'un composé de formule

$$R_1—O—CH_2—\underset{\displaystyle OH}{\underset{|}{CH}}—CH_2—X \qquad I$$

dans laquelle X est un groupe de formule

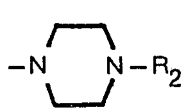 ou 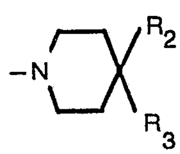

où $R_2$ est l'hydrogène, un groupe alkyle ayant 1 à 3 atomes de carbone, benzyle, benzyle monosubstitué, phényle ou phényle monosubstitué; $R_3$ est l'hydrogène ou un groupe méthyle, $—CH_2NH_2$ ou $—CONH_2$; et $R_1$ est un groupe n-alkyle de 10 à 20 atomes de carbone, benzyle ou benzyle monosubstitué, dont le substituant est un radical chloro, bromo, fluoro, alkyle ayant 1 à 3 atomes de carbone ou alkoxy ayant 1 à 3 atomes de carbone; à condition que lorsque $R_1$ est autre chose qu'un groupe alkyle, X soit un groupe 1—$R_2$-pipérazino et $R_2$ soit un groupe benzyle ou benzyle monosubstitué, caractérisé en ce qu'on chauffe un composé de formule

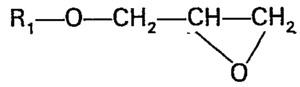

II

en présence d'une 1—$R_2$-pipérazine ou d'une 4—$R_2R_3$-pipéridine appropriée de formule HX, sous réserve que lorsque $R_3$ est le groupe —$CH_2NH_2$, un composé de formule II soit chauffé avec une 4—$R_2$-4-cyanopipéridine, l'opération étant suivie de la réduction du groupe cyano en groupe —$CH_2NH_2$;

et, le cas échéant, lorsque $R_3$ est le groupe —$CONH_2$, le composé de formule I est réduit pour former le composé de formule I dans laquelle $R_3$ est le groupe —$CH_2NH_2$;

et, le case échéant, le composé de formule I dans lequel X est le groupe 1-benzylpipérazino et $R_1$ est un groupe n-alkyle et soumis à une hydrogénolyse pour former le composé correspondant dans lequel X est le groupe pipérazino;

et, le cas échéant, lorsque X est le groupe pipérazino et $R_1$ est un groupe n-alkyle, le composé de formule I est amené à réagir avec un halogénure d'alkyle approprié ou un halogénure de benzyle substitué en présence d'une base pour former un composé de formule I dans laquelle X est un groupe 1—$R_2$-pipérazino et $R_2$ est un groupe n-alkyle ou benzyle substitué;

et, le cas échéant, à faire entrer le composé de formule I en contact avec un acide pharmaceutiquement acceptable pour former un sel d'addition d'acide de ce composé.

2. Procédé suivant la revendication 1, dans lequel le composé de formule II est chauffé avec le composé HX à une température de 75 à 250°C.

3. Procédé suivant la revendication 1, dans lequel le groupe cyano est réduit au nickel de Raney et à l'hydrogène à une température de 40 à 140°C.

4. Procédé suivant la revendication 1, dans lequel le groupe —$CONH_2$ est réduit avec un hydrure métallique à une température de 25 à 100°C.

5. Procédé suivant la revendication 1, dans lequel l'hydrogénolyse est conduite à une température de 10 à 50°C en utilisant l'hydrogène et un catalyseur au palladium fixé sur du carbone.

6. Procédé suivant la revendication 1, dans lequel ledit composé de formule I dans laquelle X est le groupe pipérazino est amené à réagir avec un halogénure de n-alkyle ou un halogénure de benzyle substitué à une température de 50 à 150°C en présence d'une trialkylamine.

**Patentansprüche für die Vertragsstaaten: BE CH LI DE FR GB IT LU NL SE**

1. Eine Verbindung der Formel

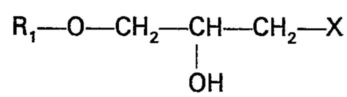

und deren pharmazeutisch annehmbare Säureadditionssalze, worin X

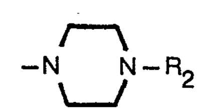 oder 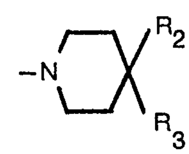

bedeutet, in denen $R_2$ Wasserstoff, Alkyl mit 1 bis 3 Kohlenstoffatomen, Benzyl, monosubstituiertes Benzyl, Phenyl oder monosubstituiertes Phenyl, wobei die genannten Substituenten Chloro, Bromo, Fluoro, Alkyl mit 1 bis 3 Kohlenstoffatomen oder Alkoxy mit 1 bis 3, Kohlenstoffatomen sind; $R_3$ Wasserstoff, Methyl, —$CH_2NH_2$ oder —$CONH_2$; und $R_1$ n-Alkyl mit 10 bis 20 Kohlenstoffatomen, Benzyl oder monosubstituiertes Benzyl, wobei besagter Substituent Chloro, Bromo, Fluoro, Alkyl mit 1 bis 3 Kohlenstoffatomen oder Alkoxy mit 1 bis 3 Kohlenstoffatomen ist, bedeuten, mit der Maßgabe, daß $R_1$ Benzyl oder monosubstituiertes Benzyl nur dann ist wenn X

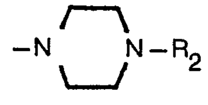 ist und

$R_2$ Benzyl oder monosubstituiertes Benzyl ist.

2. Verbindung gemäß Anspruch 1 worin X

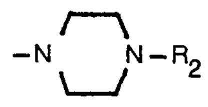 ist.

3. Verbindung gemäß Anspruch 2, worin $R_2$ Benzyl oder monosubstituiertes Benzyl ist.

4. Verbindung gemäß Anspruch 3, worin $R_1$ n-Alkyl mit 16 bis 18 Kohlenstoffatomen oder Benzyl ist.

5. Verbindung gemäß Anspruch 4, worin $R_1$ und $R_2$ jeweils Benzyl sind.

6. Verbindung gemäß Anspruch 2, worin $R_1$ n-Alkyl mit 16 Kohlenstoffatomen und $R_2$ Wasserstoff bedeuten.

7. Verbindung gemäß Anspruch 1, worin X

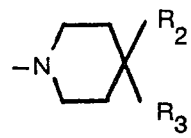

ist.

8. Verbindung gemäß Anspruch 7, worin $R_2$ Phenyl oder Wasserstoff und $R_3$ Wasserstoff, Methyl, $-CH_2NH_2$ oder $-CONH_2$ sind.

9. Verbindung gemäß Anspruch 8, worin $R_1$ n-Alkyl mit 16 bis 18 Kohlenstoffatomen ist.

10. Pharmazeutische Zusammensetzung enthaltend eine für die Immunregulierung wirksame Menge einer Verbindung einer der vorgehenden Patentansprüche und einen pharmazeutisch annehmbaren Träger.

**Patentansprüche für den Vertragsstaat: AT**

1. Verfahren zur Herstellung einer Verbindung der Formel

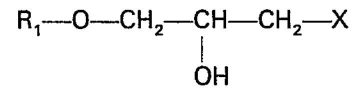

I

worin X

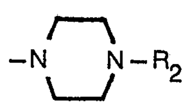

oder

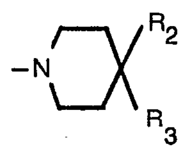

bedeutet in denen $R_2$ Wasserstoff, Alkyl mit 1 bis 3 Kohlenstoffatomen, Benzyl, monosubstituiertes Benzyl, Phenyl oder monosubstituiertes Phenyl; $R_3$ Wasserstoff, Methyl, $-CH_2NH_2$ oder $-CONH_2$; und $R_1$ n-Alkyl mit 10 bis 20 Kohlenstoffatomen, Benzyl oder monosubstituiertes Benzyl darstellen, wobei der besagte Substituent Chloro, Bromo, Fluoro, Alkyl mit 1 bis 3 Kohlenstoffatomen oder Alkoxy mit 1 bis 3 Kohlenstoffatomen ist, mit der Maßgabe, daß wenn $R_1$ eine andere Bedeutung als Alkyl besitzt X die Bedeutung von 1—$R_2$-Piperazino und $R_2$ Benzyl oder monosubstituiertes Benzyl haben, dadurch gekennzeichnet, daß eine Verbindung der Formel

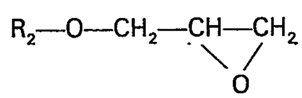

II

in Anwesenheit eines geeigneten 1—$R_2$-Piperazins oder 4—$R_3R_3$-Piperidins der Formel HX erhitzt wird, mit der Maßgabe, daß wenn $R_3$ $-CH_2NH_2$ bedeutet, eine Verbindung der Formel II mit 4—$R_2$-4-Cyanopiperidin erhitzt wird gefolgt durch Reduktion der Cyanogruppe zu $-CH_2NH_2$;

und, falls gewünscht, wenn $R_3$ $-CONH_2$ ist, die Verbindung der Formel I reduziert wird zur Bildung der Verbindung der Formel I in welcher $R_3$ $-CH_2NH_2$ ist;

und, wenn gewünscht, die Verbindung der Formel I, in welcher X 1-Benzyl-Piperazino und $R_1$ n-Alkyl darstellen, einer Hydrierung unterworfen wird zur Bildung der entsprechenden Verbindung in welcher X Piperazino ist;

und, wenn gewünscht, wenn X Piperazino und $R_1$ n-Alkyl darstellen, die Verbindung der Formel I' mit einem geeigneten Alkylhalogenid oder substituierten Benzylhalogenid in der Anwesenheit einer Base zur Umsetzung gebracht wird zur Bildung einer Verbindung der Formel I in welcher X 1—$R_2$-Piperazino und $R_2$ n-Alkyl oder substituiertes Benzyl darstellen;

und, wenn gewünscht, die Verbindung der Formel I mit einer pharmazeutisch annehmbaren Säure zusammengebracht wird zur Bildung des Säureadditionssalzes.

2. Verfahren gemäß Anspruch 1, wobei die Verbindung der Formel II mit der Verbindung HX bei einer Temperatur von 75 bis 250°C erhitzt wird.

3. Verfahren gemäß Anspruch 1, wobei die Cyanogruppe mit Raney Nickel und Wasserstoff bei einer Temperatur von 40 bis 140°C reduziert wird.

4. Verfahren gemäß Anspruch 1, wobei die $-CONH_2$-Gruppe mit einem Metallhydrid bei einer Temperatur von 25 bis 100°C reduziert wird.

5. Verfahren gemäß Anspruch 1, wobei die Hydrierung bei einer Temperatur von 10 bis 50°C unter Verwendung von Wasserstoff und einem Palladium-auf-Kohle-Katalysator durchgeführt wird.

6. Verfahren gemäß Anspruch 1, wobei die Verbindung der Formel I in welcher X Piperazino darstellt, mit einem n-Alkylhalogenid oder substituierten Benzylhalogenid bei einer Temperatur von 50 bis 150°C in Anwesenheit eines Trialkylamins umgesetzt wird.